# EUROPÄISCHE PATENTSCHRIFT

(11) **EP 4 234 210 B1**
(45) Veröffentlichungstag und Bekanntmachung des Hinweises auf die Patenterteilung: **25.12.2024**
(21) Anmeldenummer: 23152564.3
(22) Anmeldetag: 20.01.2023
(51) Int. Cl.: B29C 53/08, B29C 63/18, A61B 46/10

(54) **VERFAHREN ZUM BEARBEITEN EINES FOLIENSCHLAUCHS**
METHOD FOR PROCESSING A TUBULAR FILM
PROCÉDÉ D'USINAGE D'UN FILM TUBULAIRE

(30) Priorität: 26.01.2022 DE 102022101790
(43) Veröffentlichungstag der Anmeldung: 30.08.2023
(73) Patentinhaber: Plur, Andreas, 63571 Gelnhausen (DE)
(72) Erfinder: Plur, Andreas, 63571 Gelnhausen (DE)
(74) Vertreter: Stoffregen, Hans-Herbert

(56) Entgegenhaltungen:
- DE-U1- 202021 101 682
- US-A1- 2004 040 262

## Beschreibung

Die Erfindung betrifft ein Verfahren zum Bearbeiten eines Folienschlauchs, insbesondere einer Folienschlauchabdeckung für ein medizinisches Gerät.

Eine Vorrichtung und ein Verfahren zur Herstellung eines über seine Längserstreckung Applikationen aufweisenden schlauchförmigen Folienüberzugs zum Schutz eines medizinischen Gerätes ist aus der DE 20 2021 101 682 U1 bekannt.

Bei dem bekannten Verfahren wird in einem ersten Schritt ein ungefalteter und unbearbeiteter Folienschlauch, d.h., ein Folienschlauch, der keine Applikationen aufweist, teleskopartig gefaltet. Dabei wird ein Ende eines Folienschlauchs an zumindest eine Falthilfe angelegt, wobei die Faltung durch wiederholtes abschnittsweises Falten des Folienschlauchs gegen die Falthilfe hergestellt wird. Insbesondere wird das Ende des Folienschlauchs mit einer Lage gegen eine Ober- oder Unterseite der zumindest einen Falthilfe angelegt. Sodann wird die Lage an der zumindest einen Falthilfe fixiert. Anschließend erfolgt ein Falten der unteren bzw. oberen Lage des Schlauchs gegen einen Rand der Falthilfe zur Bildung einer ersten oder weiteren Faltung. Nach Herstellen der Faltung wird das gefaltete Ende von der zumindest einen Falthilfe abgezogen. Der Vorgang wird mehrfach wiederholt, bis die gewünschte Anzahl von Faltungen erreicht ist. Die Faltungen werden nach Vorgabe entsprechend den aufzubringenden Applikationen mit unterschiedlichen Falttiefen hergestellt. Zur Einstellung der Falttiefe sind an der Falthilfe Markierungen aufgebracht. Die Faltung kann händisch oder automatisch durchgeführt werden.

In einem zweiten Schritt wird der nach Vorgabe teleskopartig gefaltete Folienschlauch mit Applikationen versehen. Dazu wird ein Ende des gefalteten Folienschlauchs in eine Faltvorrichtung eingespannt und zumindest so weit entfaltet, bis ein Faltabschnitt erreicht ist, an dem eine Applikation anzubringen ist. In vorbestimmten Faltabschnitten, die durch die Faltungen vorgegeben sind, werden dann Applikationen an oder in eine Folienbahn appliziert.

Zum Applizieren der Applikationen kann in den entfalteten Folienschlauch eine Applikationshilfe eingebracht werden, wie diese z. B. aus der DE 20 2021 101 686 U1 bekannt ist. Der gefaltete Folienschlauch wird entweder über die Applikationshilfe gestülpt oder die Applikationshilfe wird in den Folienschlauch eingeschoben.

Gemäß der DE 2021 101 686 U1 ist vorgesehen, dass die Applikationshilfe in einen ersten, zusammengelegten Zustand bringbar ist, in dem diese eine Querschnittsfläche aufweist, die kleiner als eine Querschnittsfläche des Folienschlauchs ist, wobei die Applikationshilfe in dem ersten, zusammengelegten Zustand in den Folienschlauch einführbar bzw. der Folienschlauch über die Applikationshilfe stülpbar ist und wobei die Applikationshilfe im Inneren des Folienschlauchs in einen zweiten, die Arbeitsfläche bildenden Zustand bringbar ist, in dem die Applikationshilfe einen Querschnitt aufweist, der im Wesentlichem dem Querschnitt des aufgespannten Folienschlauchs entspricht.

Nach Anbringen der Applikationen wird der die Applikationen aufweisende Folienschlauch in einem dritten Schritt mittels der aus DE 20 2021 101 682 U1 bekannten Falteinrichtung oder händisch zurückgefaltet.

Das Verfahren umfassend die Schritte Erstfaltung des Folienschlauchs ohne Applikationen, anschließendes Entfalten des Folienschlauchs zum Aufbringen der Applikationen, Aufbringen der Applikationen auf den vorgefalteten Folienschlauch und die Rückfaltung des die Applikationen aufweisenden Folienschlauch hat den Vorteil, dass die Rückfaltung mit Applikationen aufgrund der zuvor eingebrachten Faltlinien auch für ungeübtes Personal einfach und präzise auszuführbar ist.

Allerdings wird der Folienschlauch durch die mehrfache Faltung belastet, was beim Einsatz des Folienschlauchs, z. B. zum Schutz eines OP-Roboterarms, zu Beschädigungen im Bereich der Faltlinien führen kann.

Die WO 2009/077176 A1 betrifft eine Folienschlauchabdeckung für medizinische Geräte in Form eines Folienschlauchs mit Steckfaltung. Der Folienschlauch weist einen offenen und einen geschlossenen Endabschnitt auf und eine sterile und eine nicht sterile Seite. Die Folienschlauchabdeckung weist auf einer Außenseite des geschlossenen Endabschnitts auf der nicht sterilen Seite eine Klebefläche auf, an der das medizinische Gerät befestigt wird. Im Verlauf der Steckfaltung sind keine Applikationen angebracht.

Die WO 2011/059413 A1 betrifft ein Verfahren und eine Vorrichtung zur Herstellung eines Überzugs mit teleskopartiger Faltung. Dabei wird ein Schlauch von einer Rolle abgerollt und über einen ersten Satz plattenförmiger Falthilfen gestülpt, die mit einem zweiten Satz plattenförmiger Falthilfen gegeneinander verschiebbar angeordnet sind, um die teleskopartige Faltung herzustellen. Zum Falten eines Applikationen aufweisenden Überzugs ist diese Vorrichtung nicht geeignet.

Die US 3,698,791 A betrifft einen Überzug zum Schutz eines medizinischen Gerätes wie Mikroskops. Der Überzug besteht aus einem im Wesentlichen länglichen Zuschnitt aus einem dünnen, vorzugsweise transparenten, Polymer-Folienmaterial. Aus dem flächigen Zuschnitt wird durch Verschweißen von Längsrändern ein im Wesentlichen rohrförmiges Element gebildet mit einem ersten, offenen Ende und einem zweiten Ende, welches eine oder mehrere zylindrische Verlängerungen aufweist, die so dimensioniert sind, dass diese teleskopartig in die Okulare eines Mikroskops eingreifen. Auf der Folie sind Applikationen in Form von dünnen, flexiblen länglichen Bindestreifen aus einem Material appliziert, aus dem die Folie hergestellt ist. Die Streifen sind z. B. über ein doppelseitiges Klebeband an der Außenfläche der Folie befestigt. Die Herstellung des Zuschnitts, das Applizieren der Applikationen auf den Zuschnitt und anschließendes Verschweißen des Zuschnitts zu dem röhrenförmigen Element ist zeit- und kostenintensiv. Die Schweißnaht kann Ursache für eine Undichtigkeit sein.

Die US 2004/0040262 A1 bezieht sich auf eine Vorrichtung und ein Verfahren zum Verpacken von Produkten, die unterschiedliche Größen aufweisen. Die Vorrichtung umfasst einen Förderer zum Transport der zu verpackenden Produkte von einem Produktladeabschnitt zu einem Endabschnitt einer Produktverpackungshalterung. Ferner sind Mittel zum Erfassen der Abmessungen jedes zu verpackenden Produkts vorgesehen. Die Vorrichtung umfasst außerdem Mittel zum automatischen Auswählen einer Beutelausgabevorrichtung aus der Vielzahl von Beutelausgabevorrichtungen, abhängig von den erfassten Produktabmessungen.

Gegenstand der DE 20 2020 106 630 U1 ist eine gefaltete Farbschutzfolie.

Die JP 2008037441 A bezieht sich auf eine Etikettenfolie, die in einer flachen Form gefaltet und zu einer Rolle aufgewickelt wird.

Die DE 196 03 675 A1 bezieht sich auf ein Verfahren und eine Vorrichtung zur Kontrolle von Schweißnähten von Verpackungsbehältern.

Die US 4, 176,746 bezieht sich auf eine Verpackung beutelartiger Struktur.

Nach der EP 1 373 073 B2 wird von einer Rolle ein Folienschlauch abgezogen. Dabei erfolgen Zuschnitte, um Verpackungen für eine vorgegebene Menge an Produkten zur Verfügung zu stellen.

Ein Vakuumtisch zum Zuführen von Flachmaterial ist der DE 36 30 363 A1 zu entnehmen.

Davon ausgehend liegt der vorliegenden Erfindung die Aufgabe zugrunde, ein Verfahren zum Bearbeiten eines Folienschlauchs derart weiterzubilden, dass Bearbeitungen, Applikationen und Faltungen mit hoher Präzision und materialschonend ausgeführt werden können.

Das Problem wird erfindungsgemäß gelöst durch ein Verfahren zum Bearbeiten eines Folienschlauchs, insbesondere einer Folienschlauchabdeckung für ein medizinisches Gerät, umfassend die Verfahrensschritte:
- Bereitstellen des ungefaltenen Folienschlauchs,
- Halten des ungefaltenen Folienschlauchs auf einer Applikationshilfe in Form einer Handhabungseinrichtung umfassend einer Unterlage zum Halten des Folienschlauchs und einen oberhalb des Folienschlauchs in einer X-Y-Ebene verfahrbaren Arbeitskopf als Handhabungseinheit,
- Bereitstellen zumindest einer Information für die Bearbeitung des Folienschlauchs durch die Applikationshilfe, wobei Art der Bearbeitung durch die Applikationshilfe angezeigt wird und die durch die Applikationshilfe angezeigte Position durch einen Bediener auf den Folienschlauch übertragen wird oder die angezeigte Art der Bearbeitung durch den Bediener an der angezeigten Position ausgeführt wird oder wobei die zumindest eine Information zum Bearbeiten des Folienschlauchs in der Handhabungseinrichtung gespeichert und die Bearbeitung automatisch durch die Handhabungseinrichtung gemäß der bereitgestellten Information ausgeführt wird,
- wobei die Bearbeitung ein Applizieren von Applikationen auf den ungefalteten Folienschlauch umfasst und
- wobei der ungefaltete Folienschlauch nach dem Bearbeitungsschritt des Applizierens erstmalig gefaltet wird.

Bei dem gemäß der Erfindung bearbeiteten Folienschlauch werden die Faltungen nur einmal ausgeführt. Die Faltungen können aufgrund der Markierungen mit hoher Präzision eingebracht werden. Da die Faltung nur einmal durchgeführt wird, ist das Verfahren besonders materialschonend. Ein erstes Falten des unbearbeiteten Folienschlauchs, ein zweites Falten (Entfalten) des unbearbeiteten Folienschlauchs zum Bearbeiten und ein drittes Falten (Rückfalten) des bearbeiteten Folienschlauchs, wie dies nach dem Stand der Technik üblich ist, ist nicht zwingend erforderlich. Das erstmalige Falten erfolgt erfindungsgemäß nach Applizieren der Applikationen.

Die Information zur Bearbeitung des Folienschlauchs wird erfindungsgemäß durch die Applikationshilfe bereitgestellt. Dadurch wird die anschließende händische oder maschinelle Bearbeitung des Folienschlauchs erheblich vereinfacht.

Vorzugsweise ist die zumindest eine durch die Applikationshilfe bereitgestellte Information eine Position und/oder eine Art der Bearbeitung.

Gemäß einer ersten Ausführung kann die Position und/oder Art der Bearbeitung durch die Applikationshilfe angezeigt werden, wobei die durch die Applikationshilfe angezeigte Position durch einen Bediener auf den Folienschlauch übertragen wird oder wobei die angezeigte Art der Bearbeitung durch den Bediener an der angezeigten Position ausgeführt wird. Durch die Anzeige der Informationen können Folienschläuche sehr flexibel und präzise an unterschiedlichen Positionen entsprechend der angezeigten Information bearbeitet werden.

Gemäß einer zweiten Ausführung kann die Position und/oder Art der Bearbeitung in der Applikationshilfe gespeichert sein, wobei die in der Applikationshilfe gespeicherte Position durch die Applikationshilfe auf den Folienschlauch übertragen wird oder wobei die gespeicherte Art der Bearbeitung durch die Applikationshilfe in der gespeicherten Position ausgeführt wird.

Durch das Übertragen oder Aufbringen der zumindest einen folienschlauchspezifischen Information auf den ungefalteten Folienschlauch wird gegenüber dem Stand der Technik der Vorteil erreicht, dass sowohl die Bearbeitung des Folienschlauchs an vordefinierten Positionen als auch das erstmalige Falten des bearbeiteten Folienschlauchs vereinfacht wird.

Besonders bevorzugt umfasst der Verfahrensschritt der Bearbeitung ein Markieren des Folienschlauchs zur Kennzeichnung der Position der Bearbeitung, einer Faltlinie und/oder eines Schrift- und/oder Bildzeichens, wobei das Markieren des Folienschlauchs vorzugsweise durch Drucken, Zeichnen und/oder Lasern erfolgt.

Vorzugsweise umfasst der Verfahrensschritt der Bearbeitung ein Schneiden zumindest einer Lage des Folienschlauchs, wobei das Schneiden insbesondere ein Einschneiden in die oder das Ausschneiden eines Abschnitts aus zumindest eine(n) Lage des Folienschlauchs umfasst.

Eine weitere bevorzugte Ausführung sieht vor, dass der Verfahrensschritt der Bearbeitung eine Oberflächenaktivierung in Form einer physikalischen, chemischen und/oder mechanischen Behandlung und/oder Reinigung umfasst.

Des Weiteren kann der Verfahrensschritt der Bearbeitung ein Aufbringen eines Klebemittels, insbesondere eines Klebers und/oder eines Klebebandes, wie doppelseitiges Klebeband, umfassen, wobei zuvor eine Oberflächenaktivierung erfolgen kann. Besonders bevorzugt umfasst der Verfahrensschritt des Applizierens das Applizieren einer Applikation aus der Gruppe zumindest umfassend ein Haltemittel, wie Instrumentenhalter, eine Versteifung, eine Verstärkung, einen Adapter, eine Transferöffnung (Port), einen Kabelbinder, einen Magnet, eine Markierung oder eine Handhabe an den Folienschlauch.

Der Verfahrensschritt des Applizierens umfasst vorzugsweise ein Verbinden, wie Verkleben, Verschweißen oder Klemmen und/oder Pressen der Applikation.

Besonders bevorzugt wird der Folienschlauch nach Durchführung der Bearbeitung, insbesondere nach dem Verfahrensschritt des Applizierens, entlang der markierten Faltlinien gefaltet. Die Faltung kann vorzugsweise in Form einer Teleskopfaltung oder Stauchfaltung mit einer aus der DE 20 2021 101 682 U1 bekannten Faltvorrichtung ausgeführt werden. Bei der Teleskopfaltung ist vorzugsweise vorgesehen, dass diese mit unterschiedlichen Falttiefen ausgeführt wird.

Alternativ ist vorgesehen, dass der Folienschlauch nach der Bearbeitung, insbesondere den Verfahrensschritten des Markierens, Schneidens, Oberflächenaktivierens und/oder Aufbringens des Klebemittels, jedoch vor dem Verfahrensschritt des Applizierens der Applikationen, aufgerollt wird.

Schließlich wird der Folienschlauch vor und/oder nach Durchführung der Faltung einer Qualitätskontrolle unterzogen, vorzugsweise optisch durchleuchtet. Insbesondere werden die Falttiefen der einzelnen Faltungen bzw. die Faltlinien oder Fehlstellen der Folie optisch erfasst, digital detektiert und geprüft.

Besonders hervorzuheben ist, dass als Applikationshilfe eine vorzugsweise ebene Unterlage, wie Tisch oder Brett, zum Halten des Folienschlauchs verwendet wird, wobei die zumindest eine Information auf der oder durch die Unterlage in Form von Markierungen, wie ein Maßstab oder eine Schablone, angezeigt wird. Die Markierungen können in Form von optoelektronischen Markierungen oder durch einen Leuchttisch ausgebildet sein.

Als Applikationshilfe wird eine Handhabungseinrichtung verwendet werden, umfassend eine vorzugsweise ebene Unterlage, wie Tisch, zum Halten des Folienschlauchs und eine Handhabungseinheit mit vorzugsweise wechselbaren Bearbeitungseinheiten rein beispielhaft aus der Gruppe Schneid-, Markier-, Aktivierungs-, Prüf- und/oder Klebeeinheit zum Bearbeiten des Folienschlauchs, wobei die zumindest eine Information zum Bearbeiten des Folienschlauchs in der Handhabungseinrichtung gespeichert und die Bearbeitung automatisch durch die Handhabungseinrichtung ausgeführt wird.

Als Handhabungseinrichtung kann eine X-Y-Arbeitsstation verwendet werden, wobei ein Arbeitstisch der X-Y-Arbeitsstation die Unterlage und der in X-Y-Richtung steuerbare Arbeitskopf die Handhabungseinheit bildet.

Vorzugsweise wird als Unterlage ein Vakuumtisch verwendet, wobei das Fixieren des Folienschlauchs in ausgelegter flacher Form auf dem Vakuumtisch durch Erzeugen eines Vakuums erfolgt. Die Vakuumfixierung bewirkt, dass beide Lagen des Folienschlauchs sehr flach und eben, d.h., im Wesentlichen faltenfrei auf dem Vakuumtisch aufliegen, so dass dieser für weitere Bearbeitungsschritte vorbereitet ist. Zudem ermöglicht der Vakuumtisch die Verwendung eines Folienschlauchs, da durch die Fixierung der unteren Lage gleichzeitig die obere Lage des Folienschlauchs mit fixiert wird. Ein späteres Schweißen zweier Lagen ist nicht erforderlich. Der Vakuumtisch weist vorzugsweise ein umlaufendes luftdurchlässiges Förderband, wie Vliesband, auf, mittels dessen der Folienschlauch durch Erzeugen von Vakuum fixiert und transportiert werden kann.

Weitere Einzelheiten, Vorteile und Merkmale der Erfindung ergeben sich nicht nur aus den Ansprüchen, den diesen zu entnehmenden Merkmalen - für sich und/oder im Kombination -, sondern auch aus der nachfolgenden Beschreibung von den Zeichnungen zu entnehmenden bevorzugten Ausführungsbeispielen sowie deren Erläuterungen.

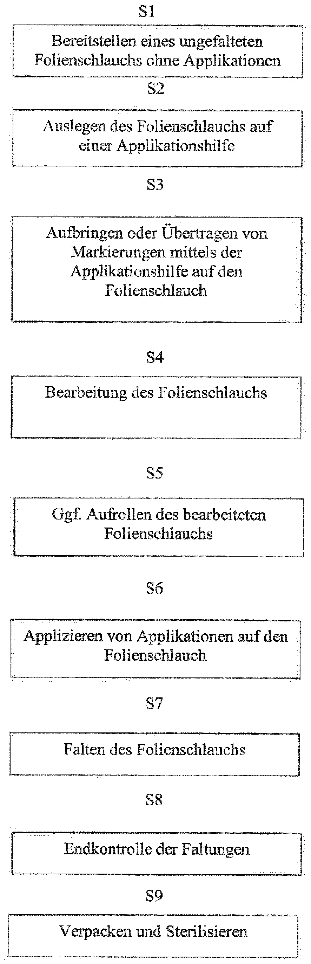

Die einzige Figur zeigt einen Verfahrensablauf zur Bearbeitung eines Folienüberzugs, wobei als Applikationshilfe eine Handhabungseinrichtung verwendet wird, umfassend eine vorzugsweise ebene Unterlage, wie Tisch, zum Halten des Folienschlauchs und eine Handhabungseinheit zum Bearbeiten des Folienschlauchs, wobei die zumindest eine Information zum Bearbeiten des Folienschlauchs in der Handhabungseinrichtung gespeichert und die Bearbeitung automatisch durch die Handhabungseinrichtung ausgeführt wird.

In einem ersten Schritt S1 wird ein ungefalteter Folienschlauch bereitgestellt. Dies kann beispielsweise durch voll automatisierte, kontinuierliche Extrusion in einem Reinraum erfolgen. Hohe Präzision und minimiertes Kontaminationsrisiko sind dabei gewährleistet. Der Folienschlauch kann kontinuierlich, vorzugsweise abgerollt von einer Rolle, oder abschnittsweise in abgelängter Form bereitgestellt werden.

In einem zweiten Schritt S2 wird der Folienschlauch flach und in gestreckter Form auf die Unterlage ausgelegt und von dieser gehalten. Die Unterlage ist ein Vakuumtisch, der oberseitig eine luftdurchlässige Schutzschicht, wie Vliesschicht, aufweist, wobei unter der Schutzschicht Luftkanäle ausgebildet sind, die mit einer Vakuumeinrichtung verbunden sind. Somit wird der Folienschlauch mittels eines Vakuums auf dem Vakuumtisch fixiert. Die Vliesschicht kann als umlaufendes Band zum Transport des Folienschlauchs ausgebildet sein.

Die Handhabungseinrichtung ist eine hochpräzise X-Y-Arbeitsstation, die einen oberhalb des Folienschlauchs in einer X-Y-Ebene verfahrbaren Arbeitskopf als Handhabungseinheit aufweist. Der Bearbeitungskopf kann ein oder mehrere Bearbeitungseinheiten in Form von Schneideinheit, Markierungseinheit, Prüfeinheit und/oder Bearbeitungseinheit zur physikalischen, chemischen und/oder mechanischen Bearbeitung aufnehmen, um den Folienschlauch zu bearbeiten.

In einem dritten Schritt S3 wird der auf dem Vakuumtisch fixierte Folienschlauch mittels der Markierungseinheit markiert. Entsprechend vorgegebener Positionen werden an einer Lage des Folienschlauchs folienspezifische Informationen in Form von Markierungen für Positionen von Faltlinien, für Positionen von Bearbeitungen und/oder weitere Informationen in Form von Schrift- oder Bildzeichen aufgebracht.

In einem weiteren Schritt S4 wird der Folienschlauch an den markierten Positionen bearbeitet. Die Bearbeitung kann Bearbeitungsschritte aus der Gruppe schneiden, wie Einschneiden, Ausschneiden und/oder Zuschneiden, eine Oberflächenaktivierung in Form einer physikalischen, chemischen und/oder mechanischen Bearbeitung, eine Reinigung, ein Aufbringen eines Klebemittels, insbesondere eines Klebers und/oder eines Klebebandes und/oder eine Qualitätsprüfung umfassen.

Mittels der Schneideinheit werden je nach Bedarf in dem ausgelegten Folienschlauch Durchbrechungen für die Applikationen eingeschnitten. Die Handhabungseinheit der hochpräzisen X-Y-Arbeitsstation kann so eingestellt sein, dass die Schneideinheit Schnitte mit einer Genauigkeit ausführt, so dass ausschließlich die obere Lage des Folienschlauchs geschnitten wird. Die Schneideinheit kann auch so eingestellt sein, dass beide Lagen in einem Schnitt geschnitten werden. Je nach Einsatzzweck werden Folien mit unterschiedlicher Materialstärke, vorzugsweise im Bereich von 40µm bis 60µm, besonders bevorzugt 55µm, verwendet. Vorzugsweise kann zum Schutz der unteren Lage eine Schutzfolie bzw. Zwischenfolie in den zu bearbeitenden Folienschlauch eingelegt werden.

Nach Durchführen der vorbereitenden Bearbeitungsschritte wie Markieren, Schneiden, Oberflächenaktivierung und/oder Auftragen von Klebemittel, wie doppelseitiges Klebeband, kann der Folienschlauch in einem weiteren, jedoch alternativen Verfahrensschritt S5, zum Transport und zur weiteren Bearbeitung aufgerollt werden.

Im Anschluss an Bearbeitungsschritt S4 oder alternativ S5 erfolgt in einem weiteren Bearbeitungsschritt S6 das Applizieren zumindest einer Applikation aus der Gruppe zumindest umfassend ein Haltemittel, wie Instrumentenhalter, eine Versteifung, eine Verstärkung, einen Adapter, eine Transferöffnung (Port), einen Kabelbinder, ein Magnet, eine Markierung, eine Handhabe an den Folienschlauch. Die Applikationen werden an den markierten Positionen entweder händisch oder automatisch appliziert.

Sowohl das Markieren gemäß Schritt S3, die Bearbeitung gemäß Schritt S4, das Aufrollen gemäß Schritt S5 als auch das Applizieren der Applikationen gemäß Schritt S6 erfolgen vorzugsweise in derselben Reinraumumgebung.

Nach dem Applizieren der Applikationen erfolgt in Schritt S7 die erstmalige Faltung des die Applikationen aufweisenden Folienschlauchs. Gemäß der Erfindung handelt es sich dabei um eine erstmalige Faltung des Folienschlauchs. Die Faltung kann händisch oder mittels einer Vorrichtung durchgeführt werden, wie diese in der Deutschen Gebrauchsmusterschrift DE 20 2021 101 682 U1 beschrieben ist. Die Faltungen werden, entsprechend den Markierungen auf dem Folienschlauch und/oder Markierungen an der Faltvorrichtung in definierten, vorzugsweise unterschiedlichen Falttiefen gefaltet.

Nach dem Falten des Folienschlauchs erfolgt in Schritt S8 optional eine Endkontrolle, wobei die verschiedenen Falttiefen mittels eines Durchleuchtungsverfahrens oder mittels optoelektronischer, digitaler Detektion vorzugsweise mittels kameragestützter Prüfung und computerbasierter Auswertung überprüft werden. Optional können weitere Bearbeitungen, wie Schweißungen und/oder Zuschnitte, ausgeführt werden. In diesem Verfahrensschritt kann z.B. eine Handhabungshilfe an einem Schlauchende angebracht werden, mittels der der gefaltete Folienschlauch z.B. auf einfache Weise über ein medizinisches Gerät, wie Roboterarm, übergezogen werden kann.

In einem Verfahrensschritt S9 erfolgt die Verpackung des die Applikationen aufweisenden Folienschlauchs und anschließende Sterilisation.

## Patentansprüche

1. Verfahren zum Bearbeiten eines Folienschlauchs, insbesondere einer Folienschlauchabdeckung für ein medizinisches Gerät, umfassend die Verfahrensschritte:
- Bereitstellen eines ungefalteten Folienschlauchs,
- Halten des ungefalteten Folienschlauchs auf einer Applikationshilfe in Form einer Handhabungseinrichtung umfassend eine Unterlage zum Halten des Folienschlauchs und einen oberhalb des Folienschlauchs in einer X-Y-Ebene verfahrbaren Arbeitskopf als Handhabungseinheit,
- Bereitstellen zumindest einer Information für die Bearbeitung des ungefalteten Folienschlauchs durch die Applikationshilfe, wobei Art der Bearbeitung durch die Applikationshilfe angezeigt wird und die durch die Applikationshilfe angezeigte Position durch einen Bediener auf den ungefalteten Folienschlauch übertragen wird oder die angezeigte Art der Bearbeitung durch den Bediener an der angezeigten Position ausgeführt wird oder wobei die zumindest eine Information zum Bearbeiten des ungefalteten Folienschlauchs in der Handhabungseinrichtung gespeichert und die Bearbeitung automatisch durch die Handhabungseinrichtung gemäß der bereitgestellten Information ausgeführt wird,
- wobei die Bearbeitung ein Applizieren von Applikationen auf den ungefalteten Folienschlauch umfasst und
- wobei der ungefaltete Folienschlauch nach dem Bearbeitungsschritt des Applizierens von Applikationen erstmalig gefaltet wird.

2. Verfahren nach Anspruch 1,
**dadurch gekennzeichnet,**
**dass** die zumindest eine Information eine Position und/oder eine Art der Bearbeitung ist.

3. Verfahren nach Anspruch 2,
**dadurch gekennzeichnet,**
**dass** die Position und/oder Art der Bearbeitung durch die Applikationshilfe angezeigt wird, wobei die durch die Applikationshilfe angezeigte Position durch einen Bediener auf den Folienschlauch übertragen wird oder wobei die angezeigte Art der Bearbeitung durch den Bediener an der Position ausgeführt wird.

4. Verfahren nach zumindest einem der vorhergehenden Ansprüche,
**dadurch gekennzeichnet,**
**dass** die Position und/oder Art der Bearbeitung in der Applikationshilfe gespeichert ist, wobei die in der Applikationshilfe gespeicherte Position durch die Applikationshilfe auf den Folienschlauch übertragen wird oder wobei die gespeicherte Art der Bearbeitung durch die Applikationshilfe in der gespeicherten Position ausgeführt wird.

5. Verfahren nach zumindest einem der vorhergehenden Ansprüche,
**dadurch gekennzeichnet,**
**dass** der Verfahrensschritt der Bearbeitung ein Markieren des Folienschlauchs zur Kennzeichnung der Position der Bearbeitung, einer Faltlinie und/oder eines Schriftund/oder Bildzeichens umfasst, wobei das Markieren des Folienschlauchs vorzugsweise durch Drucken, Zeichnen und/oder Lasern erfolgt.

6. Verfahren nach zumindest einem der vorhergehenden Ansprüche,
**dadurch gekennzeichnet,**
**dass** der Verfahrensschritt der Bearbeitung ein Schneiden zumindest einer Lage des Folienschlauchs umfasst, wobei das Schneiden insbesondere ein Einschneiden in die oder das Ausschneiden eines Abschnitts aus der zumindest eine(n) Lage des Folienschlauchs umfasst.

7. Verfahren nach zumindest einem der vorhergehenden Ansprüche,
**dadurch gekennzeichnet,**
**dass** der Verfahrensschritt der Bearbeitung eine Oberflächenaktivierung in Form einer physikalischen, chemischen und/oder mechanischen Bearbeitung und/oder Reinigung der zumindest einen Lage des Folienschlauchs umfasst.

8. Verfahren nach zumindest einem der vorhergehenden Ansprüche,
**dadurch gekennzeichnet,**
**dass** der Verfahrensschritt der Bearbeitung ein Aufbringen eines Klebemittels, insbesondere eines Klebers und/oder eines Klebebandes, wie doppelseitiges Klebeband, auf die zumindest eine Lage des Folienschlauchs umfasst.

9. Verfahren nach Anspruch 1,
**dadurch gekennzeichnet,**
**dass** die Applikation aus der Gruppe zumindest umfassend ein Haltemittel, wie Instrumentenhalter, eine Versteifung, eine Verstärkung, einen Adapter, eine Transferöffnung (Port), einen Kabelbinder, ein Magnet, eine Markierung, eine Handhabe an den Folienschlauch ausgewählt wird.

10. Verfahren nach zumindest einem der vorhergehenden Ansprüche,
**dadurch gekennzeichnet,**
**dass** der Verfahrensschritt des Applizierens ein Verbinden, wie Kleben, Schweißen, Klemmen und/oder Pressen, der Applikation an den Folienschlauch umfasst.

11. Verfahren nach Anspruch 1,
**dadurch gekennzeichnet,**
**dass** der Verfahrensschritt des Faltens eine Teleskopfaltung umfasst und dass vorzugsweise die Teleskopfaltung mit unterschiedlichen Falttiefen ausgeführt wird.

12. Verfahren nach zumindest einem der vorhergehenden Ansprüche,
**dadurch gekennzeichnet,**
**dass** der Folienschlauch nach dem Markieren, Schneiden, Oberflächenaktivieren und/oder Aufbringen der Klebefläche aufgerollt wird.

13. Verfahren nach zumindest einem der vorhergehenden Ansprüche,
**dadurch gekennzeichnet,**
**dass** der Folienschlauch vor und/oder nach Durchführung der Bearbeitungen einer Qualitätskontrolle unterzogen, vorzugsweise optisch durchleuchtet wird, wobei insbesondere nach Durchführung der Faltungen die Falttiefen der einzelnen Faltungen bzw. Faltlinien optoelektronisch erfasst, digital detektiert und geprüft werden.

14. Verfahren nach Anspruch 1,
**dadurch gekennzeichnet,**
**dass** als Applikationshilfe eine ebene Unterlage, wie Tisch, insbesondere Leuchttisch, oder Brett, zum Halten des Folienschlauchs verwendet wird, wobei die zumindest eine Information auf der oder durch die Unterlage in Form von Markierungen, wie ein Maßstab oder eine Schablone, angezeigt wird.

15. Verfahren nach Anspruch 1,
**dadurch gekennzeichnet,**
**dass** als Unterlage ein Vakuumtisch verwendet wird, wobei das Fixieren des Folienschlauchs in ausgelegter flacher Form auf dem Vakuumtisch durch Erzeugen eines Vakuums erfolgt.

## Claims

1. A method for processing a tubular film, in particular a tubular film cover for a medical device, comprising the method steps:
- Providing an unfolded tubular film,
- Holding the unfolded tubular film on an application aid in the form of a handling device, comprising a base surface for holding the tubular film and a working head, movable in an X-Y plane above the tubular film, as the handling unit,
- Providing at least one item of information for processing the unfolded tubular film by the application aid, wherein the type of processing is indicated by the application aid and the position indicated by the application aid is transferred by an operator to the unfolded tubular film, or the type of processing indicated is performed by the operator at the position indicated, or wherein the at least one item of information for processing the unfolded tubular film is saved in the handling device and processing is performed automatically by the handling device in accordance with the item of information provided,
- wherein processing comprises applying applications onto the unfolded tubular film and
- wherein the unfolded tubular film is folded for the first time after the processing step of applying applications.

2. The method according to claim 1,
**characterized in**
**that** the at least one item of information is a position and/or a type of processing.

3. The method according to claim 2,
**characterized in**
**that** the position and/or the type of processing is/are indicated by the application aid, wherein the position indicated by the application aid is transferred by an operator onto the tubular film or wherein the type of processing indicated is performed by the operator at said position.

4. The method according to at least one of the preceding claims,
**characterized in**
**that** the position and/or the type of processing is/are saved in the application aid, wherein the position saved in the application aid is transferred by the application aid onto the tubular film or wherein the type of processing saved is performed by the application aid in the position saved.

5. The method according to at least one of the preceding claims,
**characterized in**
**that** the method step of processing comprises marking of the tubular film for identification of the position of processing, of a fold line and/or of a character and/or symbol, wherein the tubular film is marked preferably by printing, drawing and/or lasering.

6. The method according to at least one of the preceding claims,
**characterized in**
**that** the method step of processing comprises cutting of at least one layer of the tubular film, wherein cutting comprises in particular cutting into the at least one layer of the tubular film or cutting out a section therefrom.

7. The method according to at least one of the preceding claims,
**characterized in**
**that** the method step of processing comprises a surface activation in the form of physical, chemical and/or mechanical processing and/or cleaning of the at least one layer of the tubular film.

8. The method according to at least one of the preceding claims,
**characterized in**
**that** the method step of processing comprises application of an adhesive means, in particular of an adhesive and/or of an adhesive tape, such as double-sided adhesive tape, onto the at least one layer of the tubular film.

9. The method according to claim 1,
**characterized in**
**that** the application is selected from the group at least comprising a holding means, such as an instrument holder, a stiffener, a reinforcement, an adapter, a transfer opening (port), a cable tie, a magnet, a marking or a handle on the tubular film.

10. The method according to at least one of the preceding claims,
**characterized in**
**that** the method step of application comprises connection, such as glueing, welding, clamping and/or pressing, of the application to the tubular film.

11. The method according to claim 1,
**characterized in**
**that** the method step of folding comprises telescopic folding, and that telescopic folding is performed preferably with differing folding depths.

12. The method according to at least one of the preceding claims,
**characterized in**
**that** the tubular film is rolled up after marking, cutting, surface activation and/or application of the adhesive surface.

13. The method according to at least one of the preceding claims,
**characterized in**
**that** the tubular film is subjected to a quality check, preferably optically screened, before and/or after performance of the processing steps, wherein in particular the folding depths of the individual folds or fold lines are optoelectronically recorded, digitally detected, and tested after performance of the folding.

14. The method according to claim 1,
**characterized in**
**that** a flat base surface such as a table, in particular a light table, or a board is used as an application aid for holding the tubular film, wherein the at least one item of information is indicated on or through the base surface in the form of markings, such as a scale or a template.

15. The method according to claim 1,
**characterized in**
**that** a vacuum table is used as the base surface, wherein the tubular film is fixed in laid-out flat form on the vacuum table by generating a vacuum.

## Revendications

1. Procédé de transformation d'un film tubulaire, notamment d'un recouvrement en forme de film tubulaire pour un appareil médical, comprenant les étapes suivantes :
- fourniture d'un film tubulaire non plié,
- maintien du film tubulaire non plié sur une aide à l'application sous la forme d'un dispositif de manipulation comprenant une surface pour maintenir le film tubulaire et une tête de travail déplaçable au-dessus du film tubulaire dans un plan X-Y comme unité de manipulation,
- fourniture d'au moins une information pour la transformation du film tubulaire non plié par l'aide à l'application, sachant que le type de transformation est affiché par l'aide à l'application et que la position affichée par l'aide à l'application est transférée sur le film tubulaire par un opérateur ou que le type de transformation affiché est exécuté par l'opérateur à la position indiquée ou sachant que ladite au moins une information pour la transformation du film tubulaire non plié est enregistrée dans le dispositif de manipulation et que la transformation est exécutée automatiquement par le dispositif de manipulation conformément à l'information fournie,
- sachant que la transformation comprend une application d'applications sur le film tubulaire non plié et
- sachant que le film tubulaire non plié est plié pour la première fois après l'étape de transformation consistant en l'application d'applications.

2. Procédé selon la revendication 1,
**caractérisé en ce**
**que** ladite au moins une information est une position et/ou un type de transformation.

3. Procédé selon la revendication 2,
**caractérisé en ce**
**que** la position et/ou le type de transformation est affiché par l'aide à l'application, sachant que la position affichée par l'aide à l'application est transférée sur le film tubulaire par un opérateur ou sachant que le type de transformation affiché est exécuté à la position par l'opérateur.

4. Procédé selon au moins l'une des revendications précédentes,
**caractérisé en ce**
**que** la position et/ou le type de transformation sont enregistrés dans l'aide à l'application, sachant que la position enregistrée dans l'aide à l'application est transférée sur le film tubulaire par l'aide à l'application ou sachant que le type de transformation enregistré est exécuté par l'aide à l'application, à la position enregistrée.

5. Procédé selon au moins l'une des revendications précédentes,
**caractérisé en ce**
**que** l'étape de transformation comprend un marquage du film tubulaire pour repérer la position de la transformation, d'une ligne de pliage et/ou d'un caractère et/ou d'une image, sachant que le marquage du film tubulaire a lieu de préférence par impression, dessin et/ou traçage au laser.

6. Procédé selon au moins l'une des revendications précédentes,
**caractérisé en ce**
**que** l'étape de transformation comprend une coupe d'au moins une couche du film tubulaire, sachant que la coupe comprend notamment une incision dans la couche ou la découpe d'une portion de ladite au moins une couche du film tubulaire.

7. Procédé selon au moins l'une des revendications précédentes,
**caractérisé en ce**
**que** l'étape de transformation comprend une activation en surface sous la forme d'un traitement physique, chimique et/ou mécanique et/ou nettoyage de ladite au moins une couche du film tubulaire.

8. Procédé selon au moins l'une des revendications précédentes,
**caractérisé en ce**
**que** l'étape de transformation comprend une pose d'un moyen adhésif, notamment d'une colle et/ou d'un ruban adhésif, tel qu'un ruban adhésif double face sur ladite au moins une couche du film tubulaire.

9. Procédé selon la revendication 1,
**caractérisé en ce**
**que** l'application est sélectionnée parmi le groupe comprenant au moins un moyen de maintien, tel qu'un porte-instrument, un raidisseur, un renfort, un adaptateur, un orifice de transfert (port), un serre-câble, un aimant, une marque, une poignée sur le film tubulaire.

10. Procédé selon au moins l'une des revendications précédentes,
**caractérisé en ce**
**que** l'étape de l'application comprend un assemblage, tel que collage, soudage, serrage et/ou compression de l'application sur le film tubulaire.

11. Procédé selon la revendication 1,
**caractérisé en ce**
**que** l'étape de pliage comprend un pliage télescopique et que de préférence, le pliage télescopique est exécuté avec différentes profondeurs de pli.

12. Procédé selon au moins l'une des revendications précédentes,
**caractérisé en ce**
**que** le film tubulaire est enroulé après le marquage, la coupe, l'activation en surface et/ou l'application de la surface adhésive.

13. Procédé selon au moins l'une des revendications précédentes,
**caractérisé en ce**
**que** le film tubulaire est soumis à un contrôle de qualité, de préférence est examiné optiquement, avant et/ou après les transformations, sachant que notamment après l'exécution des plis, les profondeurs de pliage des différents plis ou lignes de pliage sont acquises de manière optoélectronique, détectées et contrôlées numériquement.

14. Procédé selon la revendication 1,
**caractérisé en ce**
**qu'**est utilisée comme aide à l'application une surface plane, telle qu'une table, notamment une table lumineuse, ou plaque, pour maintenir le film tubulaire, sachant que ladite au moins une information est affichée sur la ou par la surface sous la forme de marquages, tels qu'une échelle ou un gabarit.

15. Procédé selon la revendication 1,
**caractérisé en ce**
**qu'**est utilisée comme surface une table à vide, sachant que la fixation du film tubulaire a lieu sous forme étalée à plat sur la table à vide par la génération d'un vide.
